# EUROPEAN PATENT SPECIFICATION

(11) **EP 0 694 313 B1**
(45) Date of publication and mention of the grant of the patent: **09.04.2003**
(21) Application number: 95110773.9
(22) Date of filing: 11.07.1995
(51) Int. Cl.: A61M 1/36, G01H 1/00

(54) **Method and equipment for monitoring of extracorporeal blood circulation devices**
Methode und Gerät zur Überwachung von extrakorporalen Blutkreislaufeinrichtungen
Méthode et équipement pour contrôler des appareils de circulation sanguine extracorporelle

(30) Priority: 21.07.1994 IT BO940349
(43) Date of publication of application: 31.01.1996
(73) Proprietor: DIDECO S.p.A., I-41037 Mirandola (Province of Modena) (IT)
(72) Inventor: Plicchi, Gianni, Bologna (IT)
(74) Representative: Porsia, Dino, Dr.

(56) References cited:
- EP-A- 0 088 627
- EP-A- 0 240 101
- WO-A-81/03702
- GB-A- 2 082 324

## Description

In modern heart surgery it is frequently necessary to operate inside the heart, which for this purpose has to be temporarily excluded from the blood circulation system. The anatomical connections to the pulmonary circulation are such that the cardiac function cannot be dissociated from the physiological gas exchange function, and consequently for open-heart surgery it is necessary to connect the patient's major blood vessels, the aorta and the vena cava, to what is known as a heart-lung machine, which can carry out the functions of pumping and exchange of oxygen and carbon dioxide for the time required for the execution of the operation.

The first generation of heart-lung machines consist of peristaltic pumps and oxygenators comprising discs which rotate partly immersed in the blood and which continuously expose a thin film of blood in an oxygen-rich environment. An important component of the machine is a heat exchanger which uses cooled or heated water as the exchange fluid, to initially induce the hypothermia necessary to lower the cardiac metabolism and then return to normal temperature. The machine is completed with auxiliary pumps and storage vessels to compensate for any loss of blood which may occur during the operation. This type of heart-lung machine can be fully dismantled, sterilized, and used more than once.

Problems of technological reliability, quality control and operation by specialist personnel have led to the appearance on the market in recent years of disposable devices for heart-lung machines, which provide all the functions described above and the corresponding connections within a few elements, most of which are plastic. In the most advanced art, even the pumping function, in addition to the functions of oxygenation and heat exchange, has been included in disposable form. Heart-lung machines of this type are described, for example, in the article "A disposable heart-lung machine" by Steven J. Phillips, ASAIO Journal, 1993 (39) - M 204-M 207, which from actual results demonstrates the possibility of integrating disposable oxygenators made by different manufacturers (Sarns, Medtronic, Terumo) with pumps which are also potentially disposable.

It is therefore to be expected that disposable machines designed for complete provision of extracorporeal circulation will be widely available on the market, the principal parts of these machines being the units for oxygenation and collection of blood from the veins and from the operation, heat exchangers and pumps.

This vast potential market for disposable heart-lung machines can be set against the present commercial reality in which oxygenators and heat-exchangers are already being produced in very large numbers in exclusively disposable form.

As the degree of integration of mechanical and physical functions in a disposable heart-lung device or machine increases, the problem of the real-time monitoring of its operation becomes more complex, especially in relation to potential problems arising from incorrect operation of the pumping units, changes in the blood coagulation characteristic and/or phenomena of interaction between the gas, the blood and the membranes or other means of oxygenation. With all this complexity of functions, there is at present no type of function monitoring which anticipates the occurrence of phenomena which may be the precursors of serious problems. For example, a failure of regulation of a peristaltic pump, regardless of whether it is of the disposable or permanent type, may result in haemolysis or incontinence. A change in the coagulation factors may reduce the working surface of the gas exchanger and lead to constriction of the arterial insertion tube, with a consequent rise of pressure in the circuits. These are mentioned for guidance, being some of the problems which may arise in such a complex system as that of a heart-lung machine, where at present it is only the result in the patient that is monitored and it is impossible to detect in advance the occurrence of anomalies which adversely affect the operation of the machine.

The invention is designed to overcome the limitations of present extracorporeal circulation devices and machines, and especially those which are wholly disposable, such as oxygenators and heat exchangers with the circuits associated with them, with the following idea for a solution. At any suitable point of the structure of such a device or machine, at least one vibration sensor is connected, with associated electronic circuits which detect certain known frequencies of the vibration spectrum characteristic of the instantaneous correct operation of the device or machine, and which compare them in real time with programmable reference values, indicating any differences to the operators in good time.

Document EP 0 088 627 disdoses an equipment for real-time monitoring of the functionality of a machine, comprising:
- means for the real-time measurement, continuously or at cyclic intervals, of the vibrations produced by the machine and to generate a proportional electrical signal;
- means for processing and comparing the electrical signals generated with one or more programmable known values indicating the correct operation of the components of the said machine;
- means for activating optical and/or acoustical signals, connected to the said processing and comparing means;
- means for electrical power supply.

According to the present invention, there is provided for an equipment of the type above disclosed adapted to operate exclusively for the real-time monitoring of the functionality of a disposable heart-lung machine, characterized by the fact that the means for activating optical and/or acoustical signals are adapted to be selectively activated to indicate which of the components of the monitored machine are producing vibrations which differ from the said programmed reference values and which can signify a malfunction of the machine.

Further characteristics of the invention, and the advantages derived therefrom, will be more clearly understood from the following description of some possible, rather than preferred, embodiments of the invention, illustrated solely by way of example and without restriction in the figures on the two attached sheets of drawings, in which
- Fig. 1 is a block diagram of a simplified embodiment of the equipment according to the invention;
- Fig. 2 is a block diagram of a more complex embodiment of the same equipment.

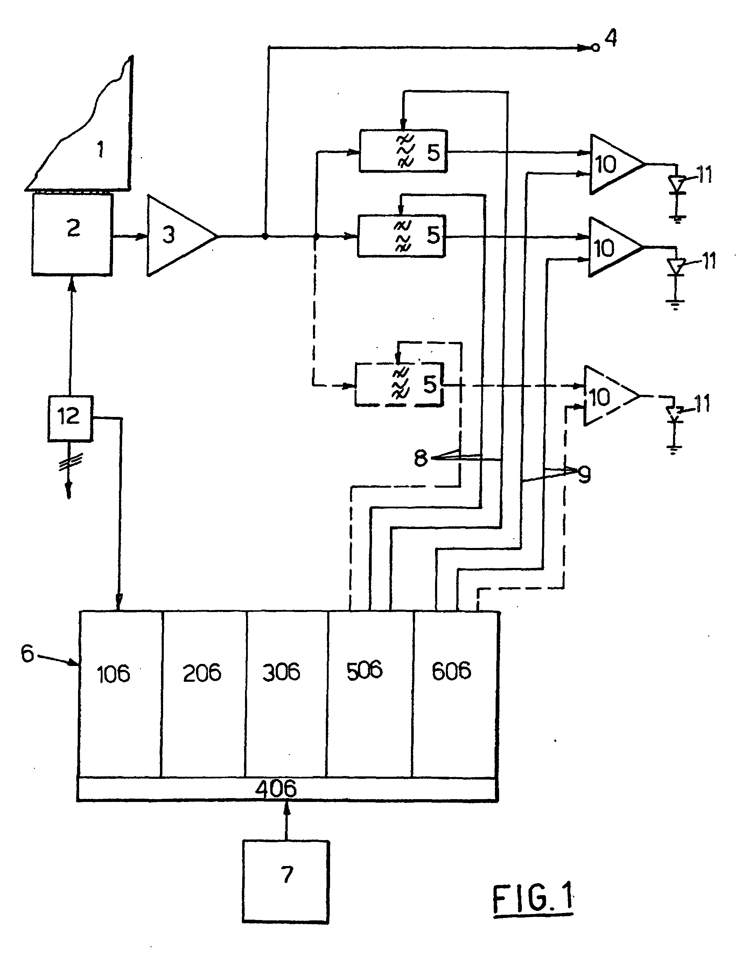

Figure 1 shows that at least one vibration transducer 2, consisting for example of an accelerometer made by any known technology or consisting of a combination of strips which form a piezoelectric pair operating in response to bending, is closely connected at any point of the structure of the machine or device 1 whose functionality is to be monitored. If a piezoelectric accelerometer, which is known to be a generator of electric charge, is used, it must be associated with a preamplifier capable of making the charge-voltage or charge-current connection. On the other hand, if a piezoresistive accelerometer is used, it will be necessary to supply it with constant voltage or constant current, detecting the variations of resistance generated in it by the applied vibration. In the diagram in Figure 1, the means of preamplification are considered to be included already in the vibration transducer 2.

The signal relating to vibration, produced by the unit 2, is amplified by a wide-band amplifier 3 characterized by a frequency response which does not alter the relevant components. For example, if the band of a wanted signal is between 0.1 and 1000 Hz, the amplifier 3 must have a constant gain at least between 0.1 and 1000 Hz.

The signal leaving the amplifier 3 is passed to an optional general analog output socket 4, which may be connected for example to a recorder and/or to an instrument for monitoring the operation of the transducer 2 and/or to provide data useful for the calibration of the equipment concerned. The output of the amplifier 3 is also connected in parallel to the inputs of one or more band-pass filters 5, which are present in a suitable quantity and have different cut-off frequencies selected from the vibration spectrum of the correct operation of the equipment or machine to be monitored, with attenuations of at least 40 dB with respect to out-of-band signals. The filters 5 are preferably of the type in which the cut-off frequencies can be programmed by means of a programmable control unit 6. If the filters 5 are made with what is known as switched-capacitance technology, for example as shown in the National Semiconductor Corporation's Linear Data Book No. 2, dated 1987, it is possible to change the cut-off frequencies of the said filters by changing the frequency of the clock signal applied to the filters. The parameter relating to the quality of the individual stages of the band-pass filters is also programmable digitally by the technology used for the said switched-capacitance filters.

If necessary, some of the said band-pass filters may also be connected in cascade, to increase further the selectivity of the selected band. This solution has not been illustrated in the drawings since it may be easily implemented from the relevant publications produced by the National Semiconductor Corporation.

The programmable controller 6 may, for example, consist of:
- a low-current microprocessor 106;
- a program memory 206;
- a data memory 306;
- an interface 406 for programming and data acquisition from an external programming device 7 which transfers data to the interface through a cable or by telemetry;
- a timing interface 506 which is used to program the clock frequencies to be sent to the filters 5 through the outputs 8;
- a digital-analog interface 606 which, through the outputs 9, supplies the analog alarm thresholds which cause the triggering of a number of comparators 10 equal to the number of the filters 5 from which the said comparators receive the vibration signals to be monitored. The trigger thresholds of the comparators 10 are also different and are selected from within the vibration spectrum of correct operation of the device or machine 1 to be monitored.

The outputs of the comparators 10 are connected, for example, to corresponding LEDs 11 which are illuminated when a signal different from the programming thresholds of the said comparators is sent from the corresponding filters 5, indicating which components of the monitored device or machine 1 are approaching or have reached a condition of incorrect operation. It is to be understood that suitable known means may be provided to make the illumination of the LEDs 11 take place intermittently. It is also to be understood that each LED 11, a red one for example, may be associated with a LED having a different colour, green for example, and that known means may be provided to make the latter LED normally illuminated when the equipment or machine to be monitored is operating correctly. Finally, it is to be understood that the outputs of the comparators may be connected to other signalling means, possibly of the acoustic type.

The number 12 schematically indicates the electrical power supply unit which enables the equipment to operate autonomously, continuously or at cyclic intervals, but always in real time.

All the electronic components 3-5-6-10 may be made with a single custom-built CMOS integrated circuit, or by means of two complementary CMOS circuits, namely one custom-built circuit containing the amplifier, filter and comparator section, and another circuit which may consist of a commercial microprocessor already provided with a program memory and data memory.

The use of CMOS technology and the active time of the heart-lung machine, which does not exceed ten hours, enable all the components of the equipment concerned, including the power supply unit 12, to be housed within the casing of the said machine or of the part of it, for example the oxygenator, which is to be monitored.

At the cost of greater complexity of the analog circuits, it is possible to add to the diagram in Figure 1 devices for calculating the effective value of vibration selected by the filters 5, making the comparators 10 operate with this signal.

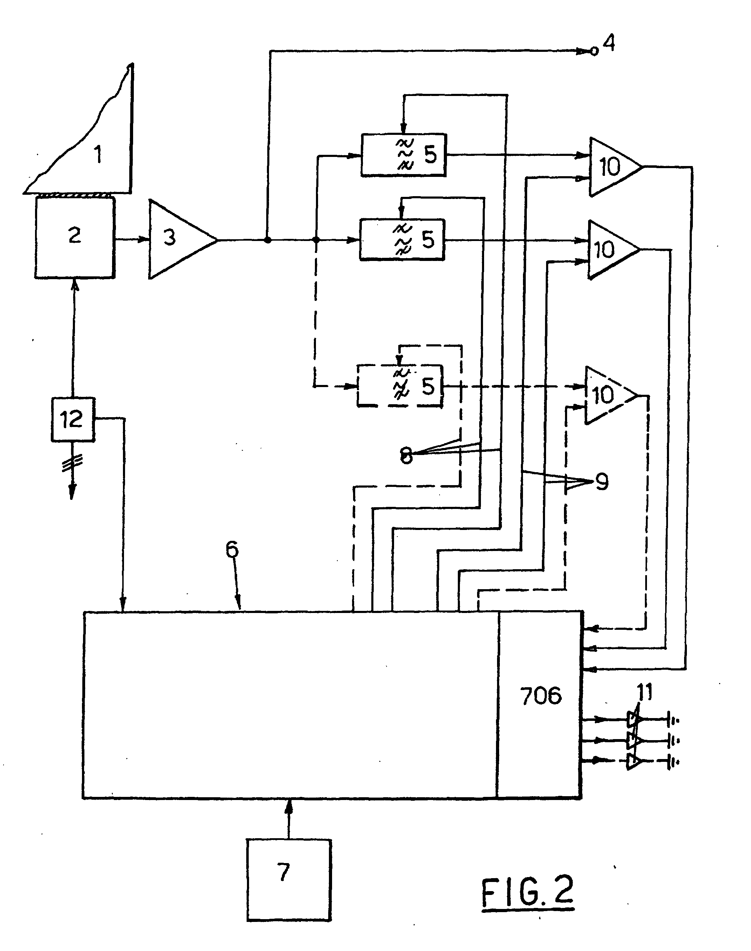

Conversely, at the cost of greater complexity of the algorithms stored in the control circuit 6, as illustrated in the diagram in Figure 2, the outputs of the comparators 10 may be connected to a circuit 706 which, according to the situations detected, determines which of the indicator lamps 11 is to be illuminated.

It is to be understood that the description refers to certain preferred embodiments of the invention, to which numerous variations and modifications, particularly as regards construction, may be made without departure from the scope of the guiding concept of the invention, as described above, as illustrated, and as claimed below. In the following claims, the references in parentheses are purely for guidance and do not limit the scope of protection of the claims.

## Claims

1. An equipment for real-time monitoring of the functionality of a disposable heart-lung machine (1), comprising:
- means (2) for the real-time measurement, continuously or at cyclic intervals, of the vibrations produced by the machine (1) and to generate a proportional electrical signal;
- means (3, 5, 6, 10) for processing and comparing the electrical signals generated with one or more programmable reference values indicating the correct operation of the components of the said machine (1);
- means (11) for activating optical and/or acoustical signals, connected to the said processing and comparing means (3, 5, 6, 10),
**characterised by** the fact that the means (11) for activating optical and/or acoustical signals are adapted to be selectively activated to indicate which of the components of the monitored machine (1) are producing vibrations which differ from the said programmed reference values and which can signify a malfunction of the machine.

2. Equipment according to Claim 1, **characterized in that** it comprises:
- at least one vibration transducer (2) of any suitable type, with a corresponding preamplifier stage, provided that it is closely connected to the casing of the device or machine to be monitored,
- at least one wide-band amplifier (3), which receives at its input the signal produced by the transducer (2) described in the preceding clause, and which has a frequency response such that it does not alter the relevant components;
- one or more band-pass filters (5), the number of which may for example be equal to the number of components of the machine or device to be monitored, with their inputs connected to the output of the amplifier (3) described in the preceding clause, and having different cut-off frequencies selected from the vibration spectrum relating to the correct operation of the device or of the components of the machine, these filters being **characterized by** a high attenuation of out-of-band signals;
- comparators (10), the number of which is at least equal to that of the said band-pass filters (5), each of which receives at one input the signal leaving a corresponding filter (5) and receives at the other input, from a programming unit (6), different threshold signals correlated and selected from the vibration spectrum relating to the correct operation of the device or of the components of the machine to be monitored;
- optical or acoustic warning devices (11) connected to the outputs of the said comparators (10) and selectively activated by the comparators when the signal from the said filters (5) is different from the programmed thresholds of the said comparators, to indicate that the device or components of the monitored machine are approaching or are in a condition of incorrect operation;
- means (12) for electrical power supply, independent of the preceding means.

3. Equipment according to Claim 2, in which the band-pass filters (5) are of what is known as the switched-capacitance type, whose cut-off frequency can be programmed by changing the frequency of the clock signal applied to the filters.

4. Equipment according to the preceding claims, in which the control and programming unit (6) used for the programming of the clock signal for the band-pass filters (5) and the programming of the trigger thresholds of the comparators (10) comprises:
- a low-current microprocessor (106);
- a program memory (206) ;
- a data memory (306);
- an interface (406) for programming and data acquisition from an external programming device (7) which transfers data to the interface through a cable or by telemetry;
- a timing interface (506) which is used to program the clock frequencies to be sent to the band-pass filters (5);
- a digital-analog interface (606) which supplies the analog trigger thresholds of the comparators (10).

5. Equipment according to the preceding claims, **characterized in that** it comprises an optional socket (4) which is connected to the output of the wide-band amplifier (3) and to which recording and/or display instruments can be connected, and which may be used for the calibration and/or monitoring of the said equipment.

6. Equipment according to the preceding claims, **characterized in that** it can be made with a single custom-built integrated CMOS circuit, or with two complementary CMOS circuits, namely one custom-built circuit comprising the amplifier, filter and comparator section, and another circuit which may consist of a commercial microprocessor already provided with a program memory and data memory, the whole of the equipment thus formed, together with the independent power supply unit (12), being made so that it can be integrated within the casing of the heart-lung machine or of the part of the machine, for example the oxygenator, which is to be monitored.

7. Equipment according to the preceding claims, **characterized in that** it comprises optional means for measuring the effective value of the signal selected by the band-pass filters (5) and **in that** it comprises means of making the comparators (10) operate with this value.

8. Equipment according to the preceding claims, **characterized in that**, in a variant embodiment, the outputs of the comparators (10) are made to be connected to a unit (706) of the control and programming circuit (6) which, according to the situations detected, determines which indicator lamp (11) is to be activated.

## Patentansprüche

1. Gerät zur Echtzeitüberwachung der Funktion einer Herz-Lungen-Maschine (1) zur Einmalverwendung, das Folgendes aufweist:
- eine Einrichtung (2) zur kontinuierlichen oder in zyklischen Intervallen erfolgenden Echtzeitmessung der Vibrationen, die von der Maschine (1) verursacht werden, und zum Erzeugen eines proportionalen elektrischen Signals;
- eine Einrichtung (3, 5, 6, 10) zum Verarbeiten und Vergleichen der erzeugten elektrischen Signale mit einem oder mehreren programmierbaren Referenzwerten, die den korrekten Betrieb der Teile der Maschine (1) anzeigen;
- eine Einrichtung (11) zum Aktivieren optischer und/oder akustischer Signale, die mit der Verarbeitungs- und Vergleichseinrichtung (3, 5, 6, 10) verbunden ist;
**dadurch gekennzeichnet, dass** die Einrichtung (11) zum Aktivieren der optischen und/oder akustischen Signale dafür ausgelegt ist, selektiv aktiviert zu werden, um anzuzeigen, welche der Teile der überwachten Maschine (1) Vibrationen verursachen, die von den programmierten Referenzwerten abweichen und die eine Fehlfunktion der Maschine bedeuten können.

2. Gerät nach Anspruch 1, **dadurch gekennzeichnet, dass** es Folgendes aufweist:
- wenigstens einen Vibrationswandler (2) irgendeines geeigneten Typs mit einer entsprechenden Vorverstärkerstufe, wobei dieser eng mit dem Gehäuse der zu überwachenden Vorrichtung oder Maschine verbunden ist,
- wenigstens einen Breitbandverstärker (3), der als Eingabe das von dem im vorigen Absatz beschriebenen Wandler (2) erzeugte Signal empfängt und dessen Frequenzgang so ist, dass er keine Änderung an den relevanten Teilen hervorruft;
- einen oder mehrere Bandbreitenfilter (5), deren Anzahl z.B. gleich der Anzahl der zu überwachenden Teile der Maschine oder Vorrichtung sein kann, deren Eingaben mit der Ausgabe des im vorigen Anspruch beschriebenen Verstärkers (3) verbunden sind und die unterschiedliche Grenzfrequenzen haben, die aus dem Vibrationsspektrum ausgewählt werden, das der korrekten Funktion der Vorrichtung oder der Teile der Maschine entspricht, wobei diese Filter durch eine starke Dämpfung von Signalen außerhalb des Bandes **gekennzeichnet** sind;
- Vergleicher (10), deren Anzahl wenigstens gleich der Anzahl der Bandbreitenfilter (5) ist, und von denen jeder als eine Eingabe das Signal empfängt, das einen entsprechenden Filter (5) verlässt und als andere Eingabe von einer Programmiereinheit (6) unterschiedliche Schwellensignale empfängt, die mit dem Vibrationsspektrum, das der korrekten Funktion der Vorrichtung oder der Teile der zu überwachenden Maschine entspricht, korrelieren und aus diesem ausgewählt sind;
- optische oder akustische Warnvorrichtungen (11), die mit den Ausgaben der Vergleicher (10) verbunden sind und selektiv von den Vergleichern aktiviert werden, wenn das Signal von den Filtern (5) von den programmierten Schwellen der Vergleicher abweicht, um anzuzeigen, dass die Vorrichtung oder Teile der überwachten Maschine in einem nicht korrekten Betriebszustand sind oder sich diesem annähern;
- eine Stromversorgungseinrichtung (12) unabhängig von den bisher genannten Einrichtungen.

3. Gerät nach Anspruch 2, bei dem die Bandbreitenfilter (5) Filter mit so genannter "geschalteter Kapazitanz" sind, deren Grenzfrequenz programmiert werden kann, indem die Frequenz des Taktsignals geändert wird, das für die Filter verwendet wird.

4. Gerät nach einem der vorigen Ansprüche, bei dem die Steuerungs- und Programmiereinheit (6), die zum Programmieren des Taktsignals für die Bandbreitenfilter (5) und zum Programmieren der Auslöseschwellen der Vergleicher (10) verwendet wird, Folgendes aufweist:
- einen Schwachstrom-Mikroprozessor (106);
- einen Programmspeicher (206);
- einen Datenspeicher (306);
- eine Schnittstelle (406) zur Programmierung und Datenerfassung von einer externen Programmiervorrichtung (7), die Daten über ein Kabel oder durch Femmessen an die Schnittstelle überträgt;
- eine Taktgeberschnittstelle (506), die verwendet wird, um die Taktfrequenzen zu programmieren, die an die Bandbreitenfilter (5) gesendet werden;
- eine Digital-Analog-Schnittstelle (606), die die analogen Auslöseschwellen der Vergleicher (10) liefert.

5. Gerät nach einem der vorigen Ansprüche, **dadurch gekennzeichnet, dass** es einen fakultativen Anschluss (4) aufweist, der mit der Ausgabe des Breitbandverstärkers (3) verbunden ist und an den Aufzeichnungs- und/oder Anzeigeinstrumente angeschlossen werden können und der zur Eichung und/oder Überwachung des Geräts verwendet werden kann.

6. Gerät nach einem der vorigen Ansprüche, **dadurch gekennzeichnet, dass** es mit einem einzigen, speziell aufgebauten integrierten CMOS-Schaltkreis oder mit zwei komplementären CMOS-Schaltkreisen, nämlich einem speziell aufgebauten Schaltkreis, der den Verstärker-, Filter- und Vergleicherabschnitt enthält, und einem weiteren Schaltkreis, der aus einem handelsüblichen, bereits mit einem Programmspeicher und einem Datenspeicher versehenen Mikroprozessor bestehen kann, ausgestattet sein kann, wobei das ganze so gebildete Gerät zusammen mit der unabhängigen Stromquelleneinheit (12) so ausgebildet ist, dass es in das Gehäuse der Herz-Lungen-Maschine oder eines zu überwachenden Teils der Maschine, z.B. der dem Oxidierer, integriert werden kann.

7. Gerät nach einem der vorigen Ansprüche, **dadurch gekennzeichnet, dass** es eine fakultative Einrichtung zum Messen des effektiven Werts des von den Bandbreitenfiltern (5) ausgewählten Signals aufweist, und dass es eine Einrichtung aufweist, die die Vergleicher (10) mit diesem Wert operieren lässt.

8. Gerät nach einem der vorigen Ansprüche, **dadurch gekennzeichnet, dass** bei einer variierenden Ausführungsform die Ausgaben der Vergleicher (10) mit einer Einheit (706) des Steuerungs- und Programmierkreises (6) verbunden sind, die gemäß den erfassten Situationen bestimmt, welche Anzeigelampe (11) zu aktivieren ist.

## Revendications

1. Equipement de contrôle en temps réel de l'aptitude à fonctionner d'une machine (1) coeur-poumons jetable, comprenant :
- des moyens (2) destinés à mesurer en temps réel, en continu ou à des intervalles cycliques, les vibrations produites par la machine (1) et à engendrer un signal électrique proportionnel ;
- des moyens (3, 5, 6, 10) destinés à traiter et à comparer les signaux électriques engendrés à une valeur de référence programmable ou à plusieurs valeurs de référence programmables indiquant le fonctionnement correct des éléments de la machine (1) ;
- des moyens (11) destinés à activer des signaux optiques et/ou acoustiques reliés aux moyens (3, 5, 6, 10) de traitement et de comparaison,
**caractérisé par le fait que** les moyens (11) destinés à activer les signaux optiques et/ou acoustiques sont conçus de façon à être activés sélectivement pour indiquer ceux des éléments de la machine (1) contrôlés qui produisent des vibrations qui diffèrent des valeurs de référence programmées et peuvent signifier un dysfonctionnement de la machine.

2. Equipement suivant la revendication 1, **caractérisé en ce qu'**il comprend :
- au moins un transducteur (2) de vibration d'un type quelconque, ayant un étage correspondant de préamplificateur, prévu de manière à être étroitement connecté au boîtier du dispositif ou de la machine à contrôler,
- au moins un amplificateur (3) à large bande, qui reçoit à son entrée le signal produit par le transducteur (2) décrit dans le paragraphe précédent et qui a une réponse en fréquence telle qu'il ne modifie pas les éléments pertinents ;
- un filtre (5) passe-bande ou plusieurs filtres (5) passe-bande, dont le nombre peut être égal, par exemple, au nombre des éléments de la machine ou du dispositif à contrôler, dont les entrées sont reliées à la sortie de l'amplificateur (3) décrit dans le paragraphe précédent, ayant des fréquences de coupure différentes choisies dans le spectre de vibration en relation avec le fonctionnement correct du dispositif ou des éléments de la machine, ces filtres étant **caractérisés par** une grande atténuation des signaux en-dehors de la bande ;
- des comparateurs (10), dont le nombre est au moins égal à celui des filtres (5) passe-bande, chacun d'entre eux recevant à son entrée le signal quittant un filtre (5) correspondant et recevant à l'autre entrée d'une unité (6) de programmation des signaux (6) différents de seuil corrélés et sélectionnés parmi le spectre de vibration en relation avec le fonctionnement correct du dispositif ou des éléments de la machine à contrôler ;
- des dispositifs (11) optiques ou acoustiques d'avertissement reliés aux sorties des comparateurs (10) et activés sélectivement par les comparateurs lorsque le signal provenant des filtres (5) est différent des seuils programmés des comparateurs, pour indiquer que le dispositif ou les éléments de la machine contrôlés tendent vers un état de fonctionnement incorrect ou sont dans un état de fonctionnement incorrect ;
- des moyens (12) d'alimentation en électricité indépendants des moyens précédents.

3. Equipement suivant la revendication 2, dans lequel les filtres (5) passe-bande sont ceux qui sont connus comme étant du type à capacité commutée, dont la fréquence de coupure peut être programmée en changeant la fréquence du signal d'horloge appliqué aux filtres.

4. Equipement suivant les revendications précédentes, dans lequel l'unité (6) de commande et de programmation utilisée pour la programmation du signal d'horloge pour les filtres (5) passe-bande et la programmation des seuils de déclenchement des comparateurs (10) comprend :
- un microprocesseur (106) à faible intensité ;
- une mémoire (206) de programme ;
- une mémoire (306) de données ;
- une interface (406) pour la programmation et l'acquisition de données à partir d'un dispositif (7) extérieur de programmation qui transfère des données à l'interface par un câble ou par télémétrie ;
- une interface (506) de minuterie qui est utilisée pour programmer les fréquences d'horloge à envoyer aux filtres (5) passe-bande ;
- une interface (606) numérique analogique qui fournit les seuils analogiques de déclenchement des comparateurs (10).

5. Equipement suivant les revendications précédentes, **caractérisé en ce qu'**il comprend une borne (4) facultative qui est reliée à la sortie de l'amplificateur (3) à bande large et à laquelle peuvent être connectés des instruments d'enregistrement et/ou d'affichage et qui peut être utilisée pour l'étalonnage et/ou pour le contrôle de l'équipement.

6. Equipement suivant les revendications précédentes, **caractérisé en ce qu'**il peut être constitué d'un circuit CMOS intégré unique dédié au client ou de deux circuits CMOS complémentaires, à savoir un circuit dédié au client comprenant l'amplificateur, le filtre et la section de comparateur et un autre circuit qui peut consister en un microprocesseur du commerce déjà muni d'une mémoire de programme et d'une mémoire de données, tout l'équipement ainsi formé ensemble avec l'unité (12) indépendante d'alimentation étant telle qu'elle peut être intégrée dans le boîtier de la machine coeur-poumons ou d'une partie de la machine, par exemple l'oxygénateur, qui doit être contrôlé.

7. Equipement suivant les revendications précédentes, **caractérisé en ce qu'**il comprend des moyens facultatifs de mesure de la valeur efficace du signal sélectionnés par les filtres (5) passe-bande et **en ce qu'**il comprend des moyens pour faire que les comparateurs (10) fonctionnent avec cette valeur.

8. Equipement suivant les revendications précédentes, **caractérisé en ce que** dans un mode de réalisation en variante, les sorties des comparateurs (10) sont connectés à une unité (706) du circuit (6) de commande et de programmation de données qui, suivant les situations détectées, détermine la lampe (11) d'indicateur qui doit être activée.
